# EUROPEAN PATENT SPECIFICATION

(11) **EP 3 752 134 B1**
(45) Date of publication and mention of the grant of the patent: **04.12.2024**
(21) Application number: 19705955.3
(22) Date of filing: 15.02.2019
(51) Int. Cl.: A61K 31/00, A61K 31/41, A61P 17/00

(54) **METHODS AND COMPOSITIONS FOR TREATING VITILIGO**
VERFAHREN UND ZUSAMMENSETZUNGEN ZUR BEHANDLUNG VON VITILIGO
PROCÉDÉS ET COMPOSITIONS PERMETTANT DE TRAITER LE VITILIGO

(30) Priority: 16.02.2018 EP 18305161
(43) Date of publication of application: 23.12.2020
(73) Proprietor: INSERM (Institut National de la Santé et de la Recherche Médicale), 75013 Paris (FR); Université Côte d'Azur, 06103 Nice Cedex 2 (FR); Centre Hospitalier Universitaire de Nice, 06200 Nice (FR)
(72) Inventor: PASSERON, Thierry, 06204 NICE CEDEX 3 (FR); TULIC, Meri, 06204 NICE CEDEX 3 (FR)
(74) Representative: Inserm Transfert
(86) International application number: PCT/EP2019/053767
(87) International publication number: WO 2019/158675

(56) References cited:
- WO-A1-99/50299
- TULIC MERI K. ET AL: "Innate lymphocyte-induced CXCR3B-mediated melanocyte apoptosis is a potential initiator of T-cell autoreactivity in vitiligo", NATURE COMMUNICATIONS, vol. 10, no. 1, 16 May 2019 (2019-05-16), XP093089275, Retrieved from the Internet <URL:https://www.nature.com/articles/s41467-019-09963-8> DOI: 10.1038/s41467-019-09963-8
- WANG X X ET AL: "Increased expression of CXCR3 and its ligands in patients with vitiligo and CXCL10 as a potential clinical marker for vitiligo", BRITISH JOURNAL OF DERMATOLOGY, OXFORD : WILEY-BLACKWELL, UK, vol. 174, no. 6, 1 June 2016 (2016-06-01), pages 1318 - 1326, XP009506920, ISSN: 0007-0963, DOI: 10.1111/BJD.14416
- WIJTMANS MAIKEL ET AL: "Towards small-molecule CXCR3 ligands with clinical potential", CHEMMEDCHEM, WILEY-VCH, DE, vol. 3, no. 6, 8 February 2012 (2012-02-08), pages 861 - 872, XP002668990, ISSN: 1860-7179, DOI: 10.1002/CMDC.200700365

## Description

### FIELD OF THE INVENTION:

The present invention is in the field of dermatology. More particularly, the invention relates to methods and compositions for treating vitiligo in a subject in need thereof.

### BACKGROUND OF THE INVENTION:

Vitiligo is an acquired depigmentation of the skin inducing a marked alteration of the quality of life of affected individuals. This disease is characterized by destruction of melanocytes that occurs mainly in the skin and results in the appearance of well circumscribed white macules. There are two types of vitiligo, i.e., segmental vitiligo located unilaterally on a segmented area of the body; and generalized vitiligo, which has more or less bilateral symmetrical spots and may become increasingly important over the years. The pathophysiological mechanisms that lead to the destruction of melanocytes in vitiligo are mainly related to an autoimmunity process (Passeron T, Ortonne JP 2005; Spritz 2007).

Vitiligo is common and affects 1% to 2% of the general population. For many patients with vitiligo, the disfigurement caused by the disease has a great impact on their quality of life (Ongenae K et al. 2006). Halting the disease progression and repigmenting the lesional skin represent the two faces of the therapeutic challenge in vitiligo. So far, none of them has been successfully addressed. Oxidative stress and immune system in genetically predisposed individuals participate to the complex pathophysiology of vitiligo. Currently, there are several therapeutic modalities that can be proposed for the treatment of vitiligo. Treatments such as narrow-band UVB (Nb-UVB), excimer light, topical steroids, topical tacrolimus or pimecrolimus and combination approaches (with phototherapy and topical steroids or calcineurin inhibitors) can provide cosmetically acceptable repigmentation (>75%) [Lepe, 2003; Ostovari, 2004; Passeron, 2004; Taieb, 2013, Dermatol Clin. 2017;35:163-170. Unfortunately, repigmentation, consisting in vitiligo skin in the differentiation and proliferation of new melanocytes, remains difficult to achieve in most cases. Some localizations, such as hands and feet, are almost impossible to fully repigment with the current approaches. In addition, it is still very difficult to compare the efficacy of different treatment modalities and the results of different studies on the same treatment because: (i) most published studies are uncontrolled; and (ii) there is not a generally accepted biometric tool to assess disease severity and response to treatment. Recently, animal models using reactive T-cells against melanocyte antigens provided interesting data on the immune reaction potentially involved in the depigmentation of vitiligo skin but this model is not adapted to study mechanisms of melanocytes differentiation and repigmentation in vitiligo skin [Mosenson, 2013][Rashighi, 2014].

In the light of limited therapies and the strong impact on quality of life of affected individuals, there is a clear need for identifying new therapeutic targets allowing their prevention, attenuation or treatment of vitiligo.

### SUMMARY OF THE INVENTION:

Any references in the description to methods of treatment refer to the compounds, pharmaceutical compositions and medicaments of the present invention for use in a method for treatment of the human body by therapy.

The present invention relates to a method for treating vitiligo in a human in need thereof comprising a step of administering to said subject a therapeutically effective amount of an antagonist of CXCR3B. In particular, the present invention is defined by claims.

### DETAILED DESCRIPTION OF THE INVENTION:

Inventors have demonstrated that in skin and blood samples obtained from subjects suffering from vitiligo the population of the innate immune system is increased (NK and ILC1 cells). When these cells are cultivated in stress condition, they produce IFNγ which induces CXCLs production, particularly, the CXCL10 production by keratinocytes and to a lesser extent by melanocytes. CXCL10 has CXCR3 as a receptor. In Human, CXCR3 has three isoforms. CXCR3A is mostly expressed on immune cells and its activation induces differentiation and proliferation. CXCR3B is only marginally expressed at the surface of immune cells and its activation induces apoptosis. For the first time, inventors have shown that melanocytes express CXCR3, more particularly CXCR3B (RNA and protein expressions). They have shown that the expression of CXCR3B on melanocytes is responsible of the initial destruction of melanocytes. This initial apoptosis of melanocyte triggers the secondary adaptive immunity against melanocytes that further destroys the remaining melanocytes. Inventors have demonstrated for the first time that siRNA of CXCR3B prevents the apoptosis of melanocytes in the presence of CXCL10. Interestingly, targeting specifically CXCR3B has the main advantage compared to pan CXCR3 antagonists or depleting or blocking antibodies, to not affect the immune cells and thus, to protect melanocyte from apoptosis without compromising the general immune response. Accordingly, the inventors have found a new target to prevent and treat vitiligo.

The disclosure relates to a method for treating vitiligo in a human in need thereof comprising a step of administering to said human a therapeutically effective amount of an antagonist of CXCR3 expressed by melanocytes.

The invention relates to a method for treating vitiligo in a human in need thereof comprising a step of administering to said human a therapeutically effective amount of an antagonist of CXCR3B.

As used herein, the terms "treating" or "treatment" refer to both prophylactic or preventive treatment as well as curative or disease modifying treatment, including treatment of subject at risk of contracting the disease or suspected to have contracted the disease as well as subject who are ill or have been diagnosed as suffering from a disease or medical condition, and includes suppression of clinical relapse. The treatment is administered to a subject having a medical disorder or who ultimately acquires the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen includes an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen employs (in part or in whole) a "loading regimen", which includes administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen employs continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term "vitiligo" refers to a condition that causes depigmentation of skin, typically in sections or patches, and affects about 1-2% of the world population. Vitiligo occurs when there is an absence of functional melanocytes (melanin-producing cells) in the skin.

As used herein, the term "subject" refers to any mammals, such as a feline, a canine, and a primate. Particularly, in the present invention, the subject is a human afflicted with or susceptible to be afflicted with vitiligo.

As used herein, the term "CXCR3" refers to Chemokine (C-X-C motif) receptor 3. Also known as G protein-coupled receptor 9 (GPR9), CD183, P-10 receptor, and Mig receptor, CXCR3 is a chemokine receptor expressed on autoreactive T cells that have been implicated in a range of physiological processes and related disorders, such as type 1 diabetes (T1 D). CXCR3 is largely absent from naive T cells but is upregulated upon activation with antigen and recruits activated cells to the sites of inflammation in response to its primary ligands: CXCL9, CXCL10, and CXCL11. β cells have been shown to predominately express CXCL10, with lower levels of CXCL9, in mouse models of T1 D (Christen et al The Journal of Immunology, 2003, 171 : 8838-6845; Orimoto et al. J Immunol 2004; 173;7017-7024; Sarkar et al. Diabetes. 2012 Feb;81 (2):436-46); and in islets from T1 D patients having insulitis (Uno et al 2010; Roep et al Clinical and Experimental Immunology, 2003, 159: 338-343; Sarkar et al. Diabetes. 2012 Feb;61 (2):438-46), In addition, T cells that have infiltrated the pancreas have been shown to express CXCR3 in T1 D mice models and type 1 diabetes patient pancreas samples (Christen et al, The Journal of Immunology, 2003, 171 : 8838-6845; Van Haiteren et al., Diabetologia 48:75-82 (2005); Uno et al 2010; Roep et al., Clinical and Experimental immunology, 2003, 159: 338-343; Sarkar et al., Diabetes. 2012 Feb;81 (2):436-46). Furthermore, knockout mice deficient in CXCR3 demonstrate a significant delay in onset of disease and a reduction in incidence of T1 D (Frigerio et al., Nature Medicine 8:1414-1420 (2002)), while overexpression of CXCL10 in the islets of transgenic mice promotes T cell infiltration and accelerates the onset of T1 D (Rhode et al., J. Immunol. 175(6): 3516-24 (2005)). CXCR3 has three isoforms in human: CXCR3A, CXCR3B and CXCR3Alt (Berchiche and Sakmar, 2016) while the isoform B is absent in rodents. As described above, the isoform CXCR3A is expressed by T lymphocytes and has an important role in the adaptive immune system (Korniejewska et al., Immunology 2011). CXCR3A activation induces differentiation and proliferation. CXCR3B is only marginally expressed at the surface of immune cells and its activation induces apoptosis. In the context of the invention, inventors target CXCR3B expressed on melanocytes obtained from vitiligo subjects. As used herein, the term "antagonist" refers to a natural or synthetic compound that has a biological effect to inhibit the activity or the expression of CXCR3B. The inhibition of activity or expression of CXCR3B prevents the destruction of melanocytes. Thus, such antagonist prevents and treats vitiligo. In a particular embodiment, the antagonist of CXCR3B is a peptide, petptidomimetic, small organic molecule, antibody, aptamers, siRNA or antisense oligonucleotide.

In a particular embodiment, the antagonist of CXCR3B is a peptide, petptidomimetic, small organic molecule, antibody, aptamers, siRNA or antisense oligonucleotide. The term "peptidomimetic" refers to a small protein-like chain designed to mimic a peptide. In a particular embodiment, the antagonist of CXCR3B is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity.

In a particular embodiment, the antagonist of CXCR3B is a small organic molecule. The term "small organic molecule" refers to a molecule of a size comparable to those organic molecules generally used in pharmaceuticals. The term excludes biological macromolecules (e.g., proteins, nucleic acids, etc.). Preferred small organic molecules range in size up to about 5000 Da, more preferably up to 2000 Da, and most preferably up to about 1000 Da.

Calbiochem has developed AS612568, an arylsulfonamide derivative that acts as CXCR3B antagonist (EMD 1205395, 4-cyano-N-(3-fluoro-4-(1H-tetrazol-5-yl)benzyl)-N-(2-fluorobenzyl)benzenesulfonamide).

In one embodiment, the antagonist of CXCR3B is the small organic molecule AS612568.

In some embodiments, the antagonist of CXCR3B is an antibody. As used herein, the term "antibody" is used in the broadest sense and specifically covers monoclonal antibodies, polyclonal antibodies, multi-specific antibodies (e.g. bispecific antibodies) formed from at least two intact antibodies, and antibody fragments so long as they exhibit the desired biological activity. The term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991,). Diabodies, in particular, are further described in EP 404, 097 and WO 93/1 1 161; whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001 ; Reiter et al., 1996; and Young et al., 1995 further describe and enable the production of effective antibody fragments. In some embodiments, the antibody is a "chimeric" antibody as described in U.S. Pat. No. 4,816,567. In some embodiments, the antibody is a humanized antibody, such as described U.S. Pat. Nos. 6,982,321 and 7,087,409. In some embodiments, the antibody is a human antibody. A "human antibody" such as described in US 6,075,181 and 6,150,584. In some embodiments, the antibody is a single domain antibody such as described in EP 0 368 684, WO 06/030220 and WO 06/003388. In a particular embodiment, the inhibitor is a monoclonal antibody. Monoclonal antibodies can be prepared and isolated using any technique that provides for the production of antibody molecules by continuous cell lines in culture. Techniques for production and isolation include but are not limited to the hybridoma technique, the human B-cell hybridoma technique and the EBV-hybridoma technique. In a particular embodiment, the antibody is specific of the isoform B of CXCR3. In some embodiments, the antibody is a single domain antibody. The term "single domain antibody" (sdAb) or "VHH" refers to the single heavy chain variable domain of antibodies of the type that can be found in Camelid mammals which are naturally devoid of light chains. Such VHH are also called "nanobody^{®}". According to the invention, sdAb can particularly be llama sdAb. Sanofi-Genzyme has developed a blocking and depleting antibody SAR440241. R&D Systems has developed a blocking antibody MAB-160. These antibodies target both CXCR3A and CXCR3B. This kind of antibodies affects mostly immune cells, notably the immune adaptive system and not melanocytes.

In one embodiment, the antagonist of CXCR3B is the antibody MAB-160.

In some embodiments, the antagonist of CXCR3B is a short hairpin RNA (shRNA), a small interfering RNA (siRNA) or an antisense oligonucleotide which inhibits the expression of CXCR3B. In a particular embodiment, the antagonist of CXCR3B expression is siRNA. A short hairpin RNA (shRNA) is a sequence of RNA that makes a tight hairpin turn that can be used to silence gene expression via RNA interference. shRNA is generally expressed using a vector introduced into cells, wherein the vector utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs that match the siRNA to which it is bound. Small interfering RNA (siRNA), sometimes known as short interfering RNA or silencing RNA, are a class of 20-25 nucleotide-long double- stranded RNA molecules that play a variety of roles in biology. Most notably, siRNA is involved in the RNA interference (RNAi) pathway whereby the siRNA interferes with the expression of a specific gene. Anti-sense oligonucleotides include anti-sense RNA molecules and anti-sense DNA molecules, would act to directly block the translation of the targeted mRNA by binding thereto and thus preventing protein translation or increasing mRNA degradation, thus decreasing the level of the targeted protein, and thus activity, in a cell. For example, antisense oligonucleotides of at least about 15 bases and complementary to unique regions of the mRNA transcript sequence can be synthesized, e.g., by conventional phosphodiester techniques. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). Antisense oligonucleotides, siRNAs, shRNAs of the invention is delivered in vivo alone or in association with a vector. In its broadest sense, a "vector" is any vehicle capable of facilitating the transfer of the antisense oligonucleotide, siRNA, shRNA or ribozyme nucleic acid to the cells and typically mast cells. Typically, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

In some embodiments, the antagonist of CXCR3B is an endonuclease. In the last few years, staggering advances in sequencing technologies have provided an unprecedentedly detailed overview of the multiple genetic aberrations in cancer. By considerably expanding the list of new potential oncogenes and tumor suppressor genes, these new data strongly emphasize the need of fast and reliable strategies to characterize the normal and pathological function of these genes and assess their role, in particular as driving factors during oncogenesis. As an alternative to more conventional approaches, such as cDNA overexpression or downregulation by RNA interference, the new technologies provide the means to recreate the actual mutations observed in cancer through direct manipulation of the genome. Indeed, natural and engineered nuclease enzymes have attracted considerable attention in the recent years. The mechanism behind endonuclease-based genome inactivating generally requires a first step of DNA single or double strand break, which can then trigger two distinct cellular mechanisms for DNA repair, which can be exploited for DNA inactivating: the errorprone nonhomologous end-joining (NHEJ) and the high-fidelity homology-directed repair (HDR).

In a particular embodiment, the endonuclease is CRISPR-cas. As used herein, the term "CRISPR-cas" has its general meaning in the art and refers to clustered regularly interspaced short palindromic repeats associated which are the segments of prokaryotic DNA containing short repetitions of base sequences.

In some embodiment, the endonuclease is CRISPR-cas9 which is from *Streptococcus* pyogenes. The CRISPR/Cas9 system has been described in US 8697359 B1 and US 2014/0068797. Originally an adaptive immune system in prokaryotes (Barrangou and Marraffini, 2014), CRISPR has been recently engineered into a new powerful tool for genome editing. It has already been successfully used to target important genes in many cell lines and organisms, including human (Mali et al., 2013, Science, Vol. 339 : 823-826), bacteria (Fabre et al., 2014, PLoS Negl. Trop. Dis., Vol. 8:e2671.), zebrafish (Hwang et al., 2013, PLoS One, Vol. 8:e68708.), C. elegans (Hai et al., 2014 Cell Res. doi: 10.1038/cr.2014.11.), bacteria (Fabre et al., 2014, PLoS Negl. Trop. Dis., Vol. 8:e2671.), plants (Mali et al., 2013, Science, Vol. 339 : 823-826), Xenopus tropicalis (Guo et al., 2014, Development, Vol. 141 : 707-714.), yeast (DiCarlo et al., 2013, Nucleic Acids Res., Vol. 41 : 4336-4343.), Drosophila (Gratz et al., 2014 Genetics, doi:10.1534/genetics.113.160713), monkeys (Niu et al., 2014, Cell, Vol. 156 : 836-843.), rabbits (Yang et al., 2014, J. Mol. Cell Biol., Vol. 6 : 97-99.), pigs (Hai et al., 2014, Cell Res. doi: 10.1038/cr.2014.11.), rats (Ma et al., 2014, Cell Res., Vol. 24 : 122-125.) and mice (Mashiko et al., 2014, Dev. Growth Differ. Vol. 56 : 122-129.). Several groups have now taken advantage of this method to introduce single point mutations (deletions or insertions) in a particular target gene, via a single gRNA. Using a pair of gRNA-directed Cas9 nucleases instead, it is also possible to induce large deletions or genomic rearrangements, such as inversions or translocations. A recent exciting development is the use of the dCas9 version of the CRISPR/Cas9 system to target protein domains for transcriptional regulation, epigenetic modification, and microscopic visualization of specific genome loci.

In some embodiment, the endonuclease is CRISPR-Cpf1 which is the more recently characterized CRISPR from Provotella and Francisella 1 (Cpf1) in Zetsche et al. ("Cpf1 is a Single RNA-guided Endonuclease of a Class 2 CRISPR-Cas System (2015); Cell; 163, 1-13).

As used herein the terms "administering" or "administration" refer to the act of inj ecting or otherwise physically delivering a substance as it exists outside the body (e.g., an antagonist of CXCR3B) into the subject, such as by mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof. In a particular embodiment, the antagonist of CXCR3B is administered topically.

A "therapeutically effective amount" is intended for a minimal amount of active agent which is necessary to impart therapeutic benefit to a subject. For example, a "therapeutically effective amount" to a subject is such an amount which induces, ameliorates or otherwise causes an improvement in the pathological symptoms, disease progression or physiological conditions associated with or resistance to succumbing to a disorder. It will be understood that the total daily usage of the compounds of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products is varied over a wide range from 0.01 to 1,000 mg *per* adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight *per* day, especially from about 0.001 mg/kg to 7 mg/kg of body weight *per* day.

The antagonists of CXCR3B as described above is combined with pharmaceutically acceptable excipients, and optionally sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. The pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These is in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polypeptide (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

In one embodiment, the antagonist of CXCR3B is administered in combination with a classical treatment of vitiligo.

As used herein, the term "classical treatment" refers to any compound, natural or synthetic, and phototherapy used for the treatment of vitiligo.

In a particular embodiment, the classical treatment refers to phototherapy, such as narrow-band UVB (Nb-UVB), PUVA, excimer laser or lamp and topical treatments.

According to the invention, compound used for the treatment of vitiligo is selected in the group consisting in: topical corticosteroids (such as clobetasol propionate, betamethasone valerate, betamethasone diproprionate, prednisolone or hydrocortisone butyrate), topical calcineurin inhibitors (such as tacrolimus or pimecrolimus), topical JAK inhibitors, topical WNT agonists, topical GSK3b inhibitors, phenylalanine, psolarens (such as oxsoralen or trisoralen) and vitamin D analogues (such as calcipotriol or tacalcitol).

As used herein, the term "GSK3b inhibitors" refers to chemical compounds that are effective in inhibiting the activity of the glycogen synthase kinase 3β. Examples of GSK3b inhibitors include hymenialdisine, dibromocantharelline, debromohymenialdisine, thiadiazolidines, thiazoles, halomethylketones, aminopyrimidines, arylindolemaleimide, metal cation (such as beryllium, lithium chloride, copper, zinc), indirubin, manzamines, meridianin, tricantin and palinurin,

As used herein, the term "WNT agonists" refers to chemical compounds that are effective in activating the WNT signaling pathways.

As used herein, the term "JAK inhibitors" refers to chemical compounds that are effective in inhibiting the activity of one or more Janus kinase enzymes (JAK1, JAK2, JAK3 and TYK2). Examples of JAK inhibitors include tofacitinib, ruxolitinib, oclacitinib, baricitinib, filgotinib, cerdulatinib, gandotinib, lestaurtinib, momelotinib, pacritinib, upadacitinib, peficitinib, fedratinib, cucurbitacin I.

A further disclosure of the present invention relates to a method of screening a drug suitable for the treatment of mitochondrial genetic diseases comprising i) providing a test compound and ii) determining the ability of said test compound to inhibit the activity of CXCR3B.

Any biological assay well known in the art could be suitable for determining the ability of the test compound to inhibit the activity of CXCR3B. In some embodiments, the assay first comprises determining the ability of the test compound to bind to CXCR3B. In some embodiments, a population of cells is then contacted and activated so as to determine the ability of the test compound to inhibit the activity of CXCR3B. In particular, the effect triggered by the test compound is determined relative to that of a population of immune cells incubated in parallel in the absence of the test compound or in the presence of a control agent either of which is analogous to a negative control condition. The term "control substance", "control agent", or "control compound" as used herein refers a molecule that is inert or has no activity relating to an ability to modulate a biological activity or expression. It is to be understood that test compounds capable of inhibiting the activity of CXCR3B, as determined using in vitro methods described herein, are likely to exhibit similar modulatory capacity in applications in vivo. Typically, the test compound is selected from the group consisting of peptides, petptidomimetics, small organic molecules, aptamers or nucleic acids. For example the test compound according to the invention is selected from a library of compounds previously synthesised, or a library of compounds for which the structure is determined in a database, or from a library of compounds that have been synthesised de novo. In some embodiments, the test compound is selected form small organic molecules.

The invention will be further illustrated by the following figures and examples. However, these examples and figures should not be interpreted in any way as limiting the scope of the present invention.

### FIGURES:

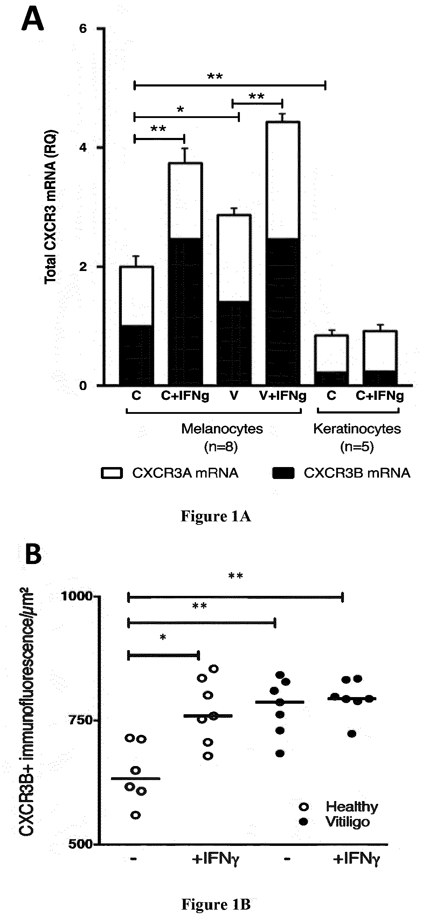

**Figure 1****. Expression of CXCR3B in human melanocytes.**
   Total CXCR3 mRNA (black+white bars) and CXCR3B mRNA (black bars) in healthy and vitiligo primary melanocytes (n=8) as well as healthy keratinocytes (n=5) before and after exposure to IFNγ (50 ng/ml) for 24 hrs (A). Results are normalised to unstimulated melanocytes from healthy, control patients and expressed as mean with SEM. (B) Semi-quantification of CXCR3B+ cells in the skin of healthy controls (open circles, n=6-7) and vitiligo patients (closed circles, n=7) prior and post stimulation. * P<0.05, **P<0.01 and ***P<0.001 versus unstimulated control.

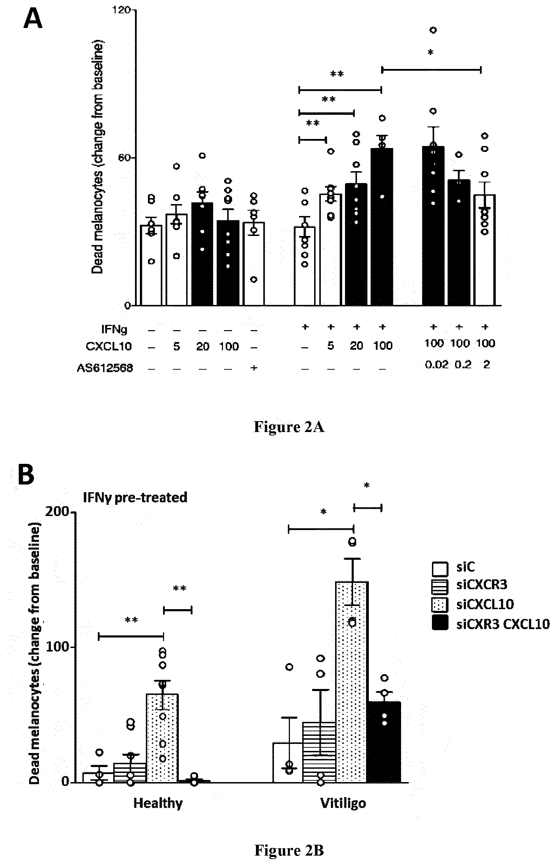

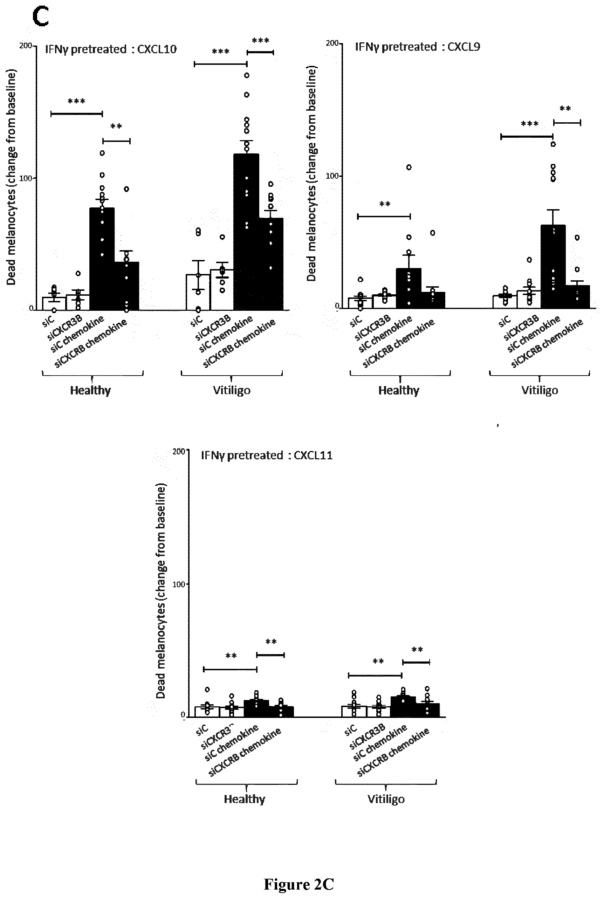

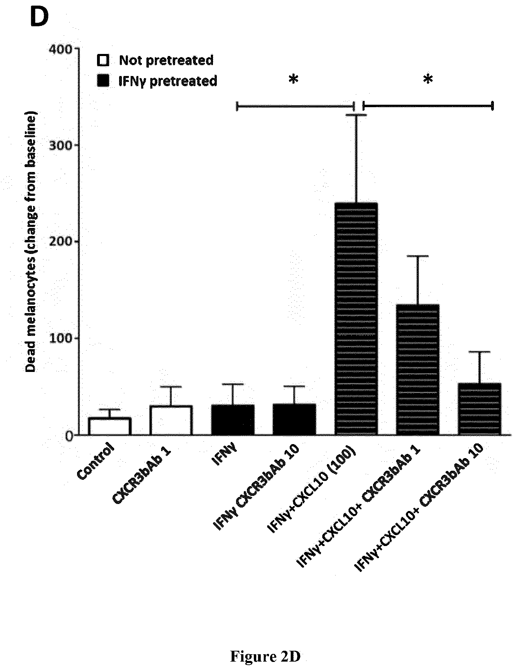

**Figure 2****: CXCL10-induced melanocyte death is driven through CXCR3B and induces apoptosis.**
   (A) Effect of CXCL10 (5, 20, 100 pg/ml) on cell viability in unstimulated or IFNγ stimulated (50 ng/ml for 48 hrs) melanocytes from vitiligo patients, in presence or absence of CXCR3 antagonist AS612568 (0.02 µM, 0.2 µM or 2 µM) (n=5-8). Cell viability was monitored using IncuCyte^{®} live cell fluorescence imaging system. (B) Effect of siCXCR3 or siC on CXCL10 (100 pg/ml)-induced death of healthy (n=4-8) and vitiligo (n=4) melanocytes. Melanocytes were tracked with CellTrackerTM Red CMPTX dye and dead cells tracked with IncuCyte^{®} Cytotox Green reagent. (C) Healthy and vitiligo melanocytes (n=6-12) were transfected with custom-designed siCXCR3B or siC and melanocyte death shown at 24 hrs following CXCL10, CXCL9 or CXCL11 (100 pg/ml) stimulation. (D) Effect of CXCL10 (100 pg/ml) on cell viability in untreated or IFNγ pretreated (50ng/ml for 48hrs) melanocytes from vitiligo patients, in presence or absence of CXCR3 blocking antibody (MAB-160, R&D Systems, USA at 1 or 10 µg/ml) (n=3). Cell viability was monitored using IncuCyte^{®} live cell fluorescence imaging system. Melanocytes were tracked with CellTrackerTM Red CMPTX dye and dead cells tracked with IncuCyte^{®} Cytotox Green reagent. Results are expressed as mean with SEM, *P<0.05, **P<0.01 and ***P<0.001.

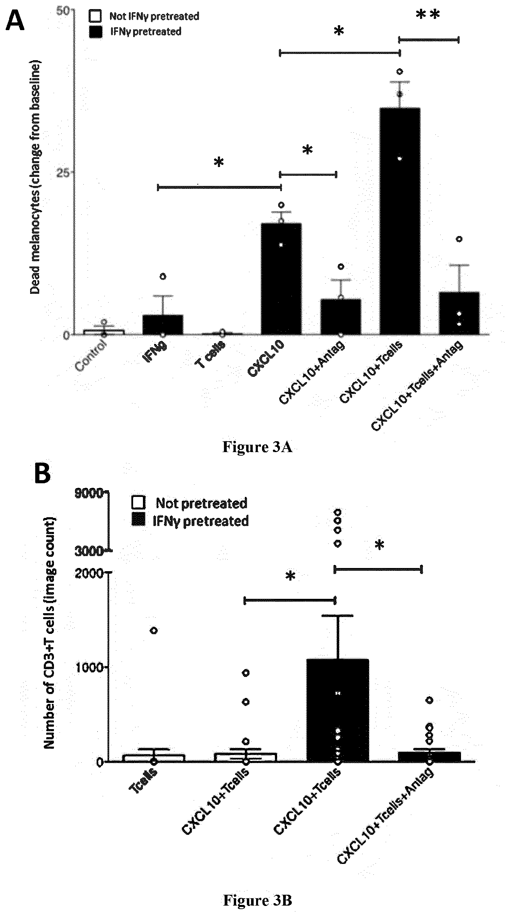

**Figure 3****: T cells enhance CXCL10-induced melanocyte death by induction of adaptive immunity**
   (A) CXCL10-induced death of vitiligo melanocytes in presence or absence of patients own autologous T cells. As above, IFNγ-pretreated melanocytes were exposed to CXCL10 in presence or absence of CXCR3 antagonist AS612568 (2 µM). The next day, media was replaced and 3 days later patient's own CD3+ T cell were sorted and added to the melanocytes (n=3) prior to initiation of IncuCyte. (B) T cell-induced potentiation of melanocyte death in IFNg-pretreated melanocytes (compared to untreated melanocytes) was associated with a parallel increase in the number of CD3+ T cells which was supported by increased expression of Ki67+ cells in the same cytospin sections.

### EXAMPLE:

### Material & Methods

### Detection of CXCR3B on human melanocytes

Melanocytes from healthy and vitiligo subjects were stimulated with 50 ng/ml IFNγ for 24 hrs and cell pellets harvested for RNA extraction. Real-time qPCR was performed using specific primers directed against CXCR3 total or against CXCR3B and results normalized to the house-keeping gene. mRNA levels were compared to healthy human keratinocytes stimulated or not with IFNγ. In separate experiments, melanocytes were grown on cover slides in 12-well plates and stimulated with IFNγ as above. 24 hours later, they were fixed at RT with 1% paraformaldehyde, saturated in PBS 3% BSA containing 3% goat serum for 1hr and incubated overnight at 4°C with primary antibody directed against CXCR3B (1:200) (Proteintech, USA). The next day, slides were washed 3x with PBS 3% BSA 0.1% Tween20 prior incubation for 1hr with secondary antibody (goat anti mouse AF594, 1:1000 at RT) and mounted with Prolong Gold Antifade reagent with DAPI (Thermofisher). The number of CXCR3B+ immunoreactive cells (red) were counted over 10 non-overlapping fields and expressed as immunofluorescence *per* mm². For in situ detection of CXCR3B+ melanocytes in the human skin, OCT frozen sections were permeabilised with PBS 0.3% TritonTM X-100 (Abcam, France) for 10 minutes prior to saturation with PBS 5% BSA 10% normal goat serum and 0.05% TritonTM X-100 for 2 hrs at RT. Slides were then incubated overnight at 4OC with primary antibodies (polylonal anti-MITF, 1:50, Sigma Aldrich and monoclonal anti-CXCR3B, 1:50, Proteintech) and the next day 1hr with secondary antibodies (anti-mouse AF594 and anti-rabbit AF488, both 1:1000) prior to mounting the slides and visualizing under confocal microscope (Nikon A1R using a 60X objective. For each slide (5 subjects per group), the number of MITF+CXCR3B+ cells (yellow immunostaining) were counted and averaged over 6 non-overlapping fields. CXCR3B staining in melanocytes was confirmed using another melanocyte marker gp100 (PMEL, premelanosome protein, 1:200, Abcam).

### Live imaging of melanocyte viability and proliferation

Melanocytes were plated in 96-well plates (10,000/well) and stimulated with 50 ng/ml IFNγ for 48 hrs prior to transfection with 50 or 80 nM siCXCR3 or siC (SmartPool scramble sequence siRNA, Dharmacon, France) or with 80nM custom-designed siCXCR3B or siC (ThermoFisher, France) using Lipofectamine^{®} RNAiMAX Reagent (Invitrogen, France) in optiMEM medium (Invitrogen). Next day, media was replaced and melanocytes stained with CellTracker^{®} Red CMPTX dye for 20 minutes at 37°C (1 µM, Molecular Probes, USA) before addition of 100 pg/ml recombinant human CXCL9, CXCL10 or CXCL11 (PeproTech) to melanocytes. Finally, Incucyte^{®} green Cytotox Reagent (100 nM, Essen Bioscience, Michigan, USA) was added to all wells and melanocyte death monitored in real-time using IncuCyte^{®} Zoom live-cell imaging system (Essen Biosciences) which was inside a 37°C humidified CO₂ incubator scanning the plate every 2 hrs. Multiple images were collected *per* well and quantification of dead melanocytes (yellow co-localised cells) was analysed using the integrated Zoom^{®} software. In separate experiments, non-transfected cells were pre-incubated with IFNγ and 48 hrs later treated with CXCR3 antagonist AS612568 (0.02, 0.2, 2 µM, Calbiochem, China) and Cytotox^{®} Reagent added just before live imaging. In other separate experiments, non-transfected cells were pre-incubated with IFNγ and 48 hrs later treated with CXCR3 blocking antibody MAB-160 (1 or 10 µg/ml, R&D Systems, USA) and Cytotox^{®} Reagent added just before live imaging To try and mimic an *in vivo* situation, vitiligo melanocytes were stimulated with IFNγ (to upregulate their CD40 and CXCR3B expression) and 48 hrs later stimulated with CXCL10 (100 pg/ml). The next day, supernatant was removed and replaced with fresh media. In separate experiments, 24 hrs post CXCL10 stimulation, all media was replaced and melanocytes left in culture for another 72 hrs before adding either *allogeneic* PBMC (positive control experiments) or *autologous* non-stimulated and sorted CD3+ T cells (1×10⁶/ml) prior to IncuCyte live imaging. At the end of the experiment (~40 hrs later), remaining melanocytes were trypsinized and co-stimulatory (CD40, HLA-DR, CD80) and adhesion cell markers (ICAM-1) on melanocytes, as well as proliferating T cells (CD3+ and Ki67+) were examined by immunofluorescence staining of cytospin sections. Monoclonal antibodies directed against human CD40 (G28.5, 1:100), HLA-DR (TU36, 1:200), CD80 (L307.4, 1:50) and ICAM-1 (HA58, 1:400) were purchased from BD Biosciences (San Diego, CA, USA). Ki67 rabbit monoclonal antibody (SP6, 1:200) was purchased from Abcam (Cambridge, UK). The number of CD3+ T cells in IncuCyte was counted from time-lapse images with Fiji software using a macro whereby a median filter was initially applied prior to application of 'Find Maxima' function to identify and count all non-stained dark spots in the images. Quantification of proliferation was performed using flow cytometry and CellTrace CFSE cell proliferation kit (Thermo Fisher Scientific, Illkirch, France). Briefly, 1×10⁶ cells were labelled with 5 µM of CFSE in 96 well plates and 72hrs later, cells collected, stained with anti-CD3 conjugated to APC (BD Biosciences, 1:100) and fluorescence measured with MACSQuant Analyzer (Miltenyi, Paris, France). Labelled cells at time zero was used as a negative reference, unstimulated cells left in culture for 72hrs before labelling as a control and cells stimulated with PHA for 72hrs (Phytohemagglutinin, 5 µg/ml) as a positive control.

### CXCL10-induced signalling in healthy and vitiligo melanocytes

Protein samples from primary melanocytes pre-stimulated with IFNγ (50 ng/ml) and incubated with CXCL9, CXCL10 or CXCL11 (100 pg/ml) for 24 or 48 hrs were extracted in buffer containing 50 mmol/L Tris-HCL (pH 7.5), 15mmol/L NaCl, 1% Triton X-100 and 1x protease and phosphatase inhibitors. Cell lysates (30 mg) were run on SDS-polyacrylamide gel and transferred to a polyvinylidene difluoride membrane (Millipore Corp). The membranes were incubated with anti-caspase 3, anti-phospho-p38, anti-phospho-ERK, anti-cleaved poly ADP-ribose polymersase (PARP) or anti-HSP90 (Cell Signalling Technology, 1:1000) followed by peroxidase-linked secondary antibodies. Reactive bands were detected using chemiluminescent substrate (Pierce). Staurosporine (1 mg/ml for 6 hr) was used as a positive control.

### Statistical analyses:

Statistical analyses were performed with Graphpad Prism^{®} 6 software. Mann-Whitney non-parametric analysis was used to test unpaired differences between groups and Wilcoxon signed rank test for paired differences. Differences were considered significant at P<0.05

### Results

### CXCR3B is expressed on human melanocytes and its regulated by IFNy

CXCR3, a chemokine CXCL9, CXCL10 and CXCL11 receptor, is typically found on T cells. Their expression on human melanocytes is unknown. Here we demonstrate that healthy human melanocytes express CXCR3B (Figure 1). This is detected at mRNA (Figure 1A) and protein (Figure 1B) level. Melanocytes from vitiligo patients have significantly elevated expression of this receptor at baseline compared to healthy controls (P<0.05). IFNγ significantly upregulates CXCR3B mRNA expression in both healthy (P<0.01) and vitiligo patients (P<0.01) (Figure 1A). However semi-quantitative analysis of CXCR3B+ immunoreactive cells demonstrate that while in the healthy skin IFNγ increases the number of CXCR3B+ cells (P<0.05, figure 1B) in vitiligo patients where this expression is already high, IFNγ did not further increase CXCR3B numbers (Figure 1B). Expression of CXCR3B mRNA in healthy human keratinocytes is significantly lower than the expression in healthy melanocytes (P<0.01) and their expression is unaffected by IFNγ treatment (Figure 1A). There is an increased number of CXCR3B+ melanocytes in the non-lesional skin of vitiligo patients compared to healthy skin (data not shown).

### CXCL10-mediated activation of CXCR3B on melanocytes drives their apoptosis

To examine the function of CXCR3 on viability of human vitiligo melanocytes, real-time detection of melanocyte death was monitored before and after exposure to CXCL10 using IncuCyte^{®} live cell imaging system. Stimulation with CXCL10 significantly increased melanocyte death compared to non-stimulated melanocytes in a dose-dependent manner (P=0.006, Figure 2A). Pre-treatment with IFNγ was required for this CXCL10-induced death. Our results have shown that in IFNγ pre-treated, siControl transfected cells, CXCL10 significantly increased the death of melanocytes (data not shown). This death was seen in melanocytes extracted from both healthy (P=0.008) and NL vitiligo skin (P=0.03) (Figure 2B). Vitiligo melanocytes were significantly more sensitive to CXCL 10-induced death compared to healthy melanocytes (P=0.004), inducing ~2-fold difference in rate of melanocyte death (Figure 2B). Transfection of melanocytes with siCXCR3 prior to CXCL10 stimulation, completely inhibited the CXCL10-induced death and restored baseline responses in both healthy (P=0.004) and vitiligo (P=0.03) melanocytes (data not shown). To more specifically examine the contributing role of CXCR3B, we repeated this experiment in both healthy and vitiligo melanocytes using custom design specific silencer RNA directed against the CXCR3B isoform. Transfection of melanocytes with siCXCR3B prior to CXCL10 stimulation, significantly reduced the CXCL10-induced death in both healthy (P=0.002) and vitiligo (P=0.001) melanocytes (Figure 2C). In addition to CXCL10, we examined responses to CXCL9 and CXCL 11. Treatment with CXCL9 and CXCL 11 also induced a significant melanocyte death in both healthy (P=0.034 and P=0.007, respectively) and vitiligo (P=P<0.001 and P=0.01) melanocytes that was almost completely prevented by the use of SiCXCR3B (Figure 2C). Interestingly, CXCL9-induced melanocyte death was significantly lower compared with CXCL10-induced death in both healthy (P<0.001) and vitiligo (P=0.0035) patients. The difference was even more pronounced with CXCL11, that induced much lower cell death compared to CXCL10 (P<0.001 for both healthy and vitiligo).

We have shown that in melanocytes extracted from healthy patients and not pre-treated with IFNγ, only a slight activation of p38 but not PARP cleavage was seen following chemokine stimulation (data not shown). This was in contrast to vitiligo patients where we observed increased activation of p38 and PARP cleavage. Studying the signalling pathway involved in chemokine-induced CXCR3B activation, p38, ERK and PARP were specifically activated by CXCL10 but not CXCL9 or CXCL11 compared to healthy controls and responses were of similar magnitude to that seen following stimulation with Staurosporin 1µg/ml (positive control). Responses were increased at 24 hours and maintained for up to 48 hrs post stimulation. Expression of total p38 and total ERK remained unchanged. Interestingly, the absence of CXCL10-induced apoptosis in normal human melanocyte without IFNγ pre-treatment (data not shown) while a significant apoptosis is observed when they are pre-treated with IFNγ (Figure 2B), is in accordance with the low basal expression of CXCR3B in normal human melanocyte and its increased expression after IFNγ stimulation (Figure 1B).

### CXCR3B-induced melanocyte death triggers the initial adaptive melanocyte autoimmunity and subsequent T cell proliferation

In live-imaging Incucyte^{®} system, melanocytes extracted from vitiligo patients and transfected with siC vector were significantly more sensitive to death compared to melanocytes extracted from healthy subjects transfected with siC (P<0.01, Figure 2B). Our IncuCyte^{®} results have shown that there was significantly higher melanocyte death when T cells were present with CXCL10-stimulated melanocytes compared to melanocyte death seen with CXCL10 stimulation alone (P=0.02) or addition of T cells alone (P=0.02) (Figure 3A). Interestingly, pre-incubation of T cells with CXCL10 for 24hrs, prior to their addition to IFNγ-primed melanocytes did not induce melanocyte death while the same T cells added to IFNγ-primed melanocytes treated with CXCL10 did suggesting lack of direct effect of CXCL10 on the T cells (data not shown). T cell enhanced melanocyte death in IFNγ-primed melanocytes was accompanied by increased melanocyte expression of co-stimulatory (CD40, CD80, HLA-DR) and adhesion (ICAM-1) molecules (data not shown) and parallel increase in absolute number of CD3+ T cells in IncuCyte co-cultures with time (P=0.03) (Figure 3B). These CD3+ T cells were indeed shown to be Ki67+ proliferating cells (data not shown). These data are supported by flow cytometric quantification of T cell proliferation which showed an increased number of dividing T cells when they were co-cultured with IFNγ-primed NHM stimulated with CXCL10 and responses were similar to that seen following PHA stimulation (data not shown). This degree of proliferation was not seen when T cells were co-cultured with non-primed NHM exposed to CXCL10. Treatment with CXCR3 antagonist AS612568 prevented the potentiating effect of T cells on CXCL10-induced melanocyte death (P=0.008) (Figure 3A), augmentation of T cell number (P=0.04) (Figure 3B) and T cell proliferation (data not shown).

In both healthy and vitiligo melanocytes, CXCL10 significantly increased the number of dead melanocytes (figure 2B) and transfection of these cells with siCXCR3B prior to CXCL10 stimulation, significantly inhibits the CXCL10-induced cell death and restores responses to baseline. CXCR3 antagonist AS612568 and CXCR3 blocking antibody CXCR3bAb inhibited melanocyte death induced by CXCL10 in a dose-dependent manner (Figure 2A and Figure 2D).

### REFERENCES:

Throughout this application, various references describe the state of the art to which this invention pertains.

A double-blind randomized trial of 0.1% tacrolimus vs 0.05% clobetasol for the treatment of childhood vitiligo; Lepe V et al, 2003; Arch Dermatol. 2003 May;139(5):581-5.

Topical tacrolimus and the 308-nm excimer laser: a synergistic combination for the treatment of vitiligo. Passeron T et al 2014 ; Arch Dermatol. 2004 Sep; 140(9):1065-9.

Guidelines for the management of vitiligo: the European Dermatology Forum consensus. Taieb A et al 2013; Br J Dermatol. 2013 Jan;168(1):5-19. doi: 10.1111/j.1365-2133.2012.11197.x. Epub 2012 Nov 2.

Medical and Maintenance Treatments for Vitiligo; Passeron T et al 2017; Dermatol Clin. 2017;35:163-170.

## Claims

1. An antagonist of CXCR3B for use for treating vitiligo in a human in need thereof.

2. The antagonist of CXCR3B for use according to claim 1, wherein, the antagonist of CXCR3B is a small organic molecule.

3. The antagonist of CXCR3B for use according to claim 1, wherein, the antagonist of CXCR3B is an antibody.

4. The antagonist of CXCR3B for use according to claim 1, wherein, the antagonist of CXCR3B expression is siRNA.

## Patentansprüche

1. Antagonist von CXCR3B zur Verwendung für die Behandlung von Vitiligo bei einem behandlungsbedürftigen Menschen.

2. Antagonist von CXCR3B zur Verwendung nach Anspruch 1, wobei der Antagonist von CXCR3B ein kleines organisches Molekül ist.

3. Antagonist von CXCR3B zur Verwendung nach Anspruch 1, wobei der Antagonist von CXCR3B ein Antikörper ist.

4. Antagonist von CXCR3B zur Verwendung nach Anspruch 1, wobei der Antagonist von CXCR3B-Expression siRNA ist.

## Revendications

1. Antagoniste de CXCR3B destiné à une utilisation pour traiter le vitiligo chez un humain qui en a besoin.

2. Antagoniste de CXCR3B destiné à une utilisation selon la revendication 1, dans lequel l'antagoniste de CXCR3B est une petite molécule organique.

3. Antagoniste de CXCR3B destiné à une utilisation selon la revendication 1, dans lequel l'antagoniste de CXCR3B est un anticorps.

4. Antagoniste de CXCR3B destiné à une utilisation selon la revendication 1, dans lequel l'antagoniste de l'expression de CXCR3B est l'ARNsi.
